# EUROPEAN PATENT APPLICATION

(11) **EP 1 698 295 A1**
(43) Date of publication of application: **06.09.2006**
(21) Application number: 06003600.1
(22) Date of filing: 22.02.2006
(51) Int. Cl.: A61B 17/86, A61B 17/88

(54) **Bone screw and driver system**

(30) Priority: 01.03.2005 US 67897
(71) Applicant: LINVATEC CORPORATION, Largo, Florida 33773 (US)
(72) Inventor: Ilomäki, Jouko, 37100 Nokia (FI); Lähteenkorva, Kimmo, 33720 Tampere (FI)
(74) Representative: Beetz & Partner

(57) **Abstract**

A low profile bioabsorbable bone screw (10) and driver (100) system. The driver (100) has a distal end (130) that partially encases the periphery (96) of the screw head (20) of the bone screw (10) and has a metal stabilizing projection (160) configured to be received by a recess (40) of the screw head (20) and to prevent the screw head (20) from laterally disengaging from the distal end (130) of the driver (100).

## Description

### FIELD OF THE INVENTION

The present invention relates to a bioabsorbable bone screw and a driver for stably inserting the bone screw into bone.

### BACKGROUND OF THE INVENTION

In the past, bone screws primarily have been made of stainless steel and titanium as such materials provide high strength and torsion resistance. A disadvantage of such bone screws is that they must be removed from the body after the healing process, thereby necessitating another surgical procedure. Bone screws made of plastic materials, particularly resorbable plastic materials, are resorbed into the body after the healing process, thereby avoiding the need of another surgical procedure to remove the bone screws from the body. Such plastic screws, however, have substantially lower strength than steel or titanium screws and there is a danger that the screw heads of such screws can torsionally shear off when the screws are turned into the bone by a screwdriver.

Because of the relatively weak strength of resorbable bone screws, the screw heads have been manufactured to have a relatively thick dimension in a direction parallel to the rotational axis of the screw. The thicker screw heads provide a greater surface area for application of torque by the driver.

Also, many conventional screw head designs for plastic screws have domed or rounded shapes with a single slot or cruciform slot. Such screws require a certain minimum thickness because the material removed from the screw head by the formation of the slots makes the screw head weaker and additional thickness is needed to make the screw heads strong enough to accept the necessary torque to drive the screw. Additionally, the single slot or cruciform slot patterns result in generally radially expanding forces applied to the screw head by the screw driver. Such expanding forces also require extra material in the screw head to enable the screw to be driven.

The relatively thick screw head of the above-described bone screws, however, can undesirably protrude from the surface within which the screw is mounted, thereby potentially causing irritation to surrounding tissue. A need therefore exist for a resorbable low profile bone screw that can withstand torsional stress.

### SUMMARY OF THE INVENTION

The present invention provides a bone screw and a driver system. In an embodiment, the bone screw of the system is a bioabsorbable bone screw having a screw head, which has a top surface integral with a periphery. The top surface defines a recess and the periphery has opposing first and second side surfaces facing away from each other. The driver of this embodiment of the system of the present invention has a distal end comprising opposing first and second side faces facing towards each other that are mutually configured to partially encase the periphery of the screw head. The distal end of the driver also comprises a metal stabilizing projection positioned between the opposing first and second side faces. The metal stabilizing projection is configured to be received by the recess of the bone screw.

### BRIEF DESCRIPTION OF THE DRAWINGS

The present invention will become more fully understood from the detailed description given hereinbelow and the accompanying drawings which are given by way of illustration only and wherein:

**FIG. 1** is a perspective view of a bone screw according to an embodiment of the present invention.

**FIG. 1a** is a top view of a bone screw according to an embodiment of the present invention.

**FIG. 2** is a side view of a bone screw according to an alternative embodiment of the present invention.

**FIG. 3** is a perspective view of a bone screw according to an alternative embodiment of the present invention.

**FIG. 3a** is a side view of the bone screw illustrated in **FIG. 3.**

**FIG. 4** is a driver according to an embodiment of the present invention.

FIG. 5 is a perspective view of driver and bone screw system according to an embodiment of the present invention.

**FIG. 6a-c** depicts use of a bone screw and driver system according to an embodiment of the present invention.

**FIG. 7** is cross-sectional of a driver according to an embodiment of the present invention.

### DETAILED DESCRIPTION OF THE INVENTION

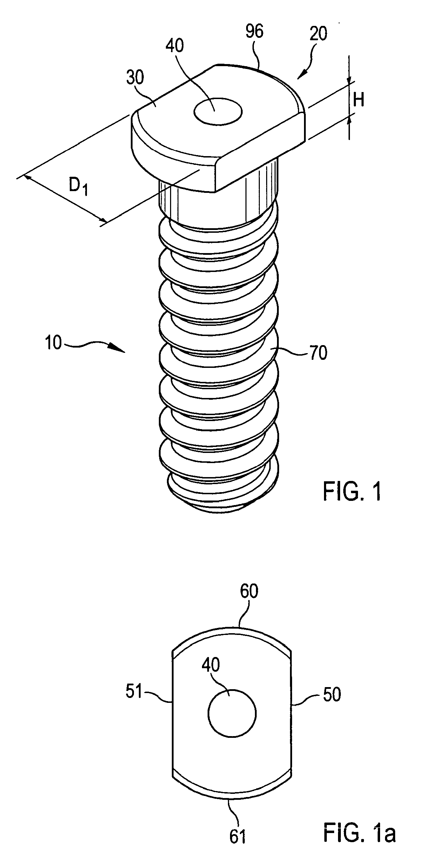

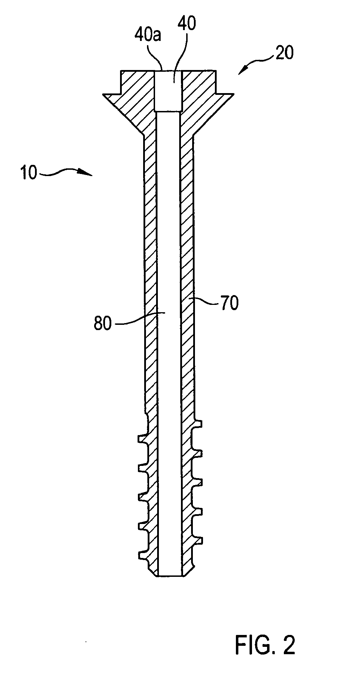

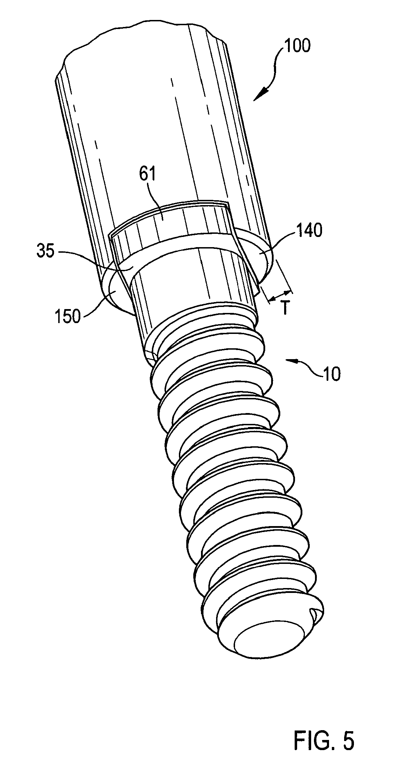

The present invention provides a bioabsorbable bone screw and driver system. Referring to **FIG. 1**, in an embodiment, the bioabsorbable bone screw **10** has a screw head **20** and an at least partially threaded shank **70** extending distally from screw head **20.** According to the present invention, screw head **20** has a top surface 30 that defines a recess **40** and a bottom surface **35** (illustrated in **FIG. 5).** Although the top plan contour of recess **40** is illustrated in **FIG.** 1 as being circular, the recess can have any top plan contour, such as circular, elliptical, rectangular, or triangular. Further, referring to **FIG. 2,** shank **70** may define a bore **80** extending from recess **40**. Although **FIG. 2** illustrates the bore extending the entire length of shank **70**, the bore may extend any distance from recess **40**. Preferably, the area of recess **40** taken at the exposed entry orifice **40a** at top surface **30** of screw head **20** is between about 0.2 mm² and 3.1 mm² and more preferably is about 0.385 mm². In embodiments where recess **40** has a circular top plan contour, the diameter of recess **40** taken at exposed entry orifice **40a** is between about 0.5 mm and 2.0 mm and more preferably is about 0.70 mm. Preferably, the depth of the recess is between approximately 0.5 mm and 2.0 mm and more preferably is about 0.8 mm. Preferably, the volume of the recess is between about 0.1mm³ and 6.2mm³. In preferred embodiments, the diameter of the recess is at least equal to the depth of the recess. Preferably, the major thread diameter of the shaft of screw having a recess with these dimensions is between about 1 mm to about 10 mm.

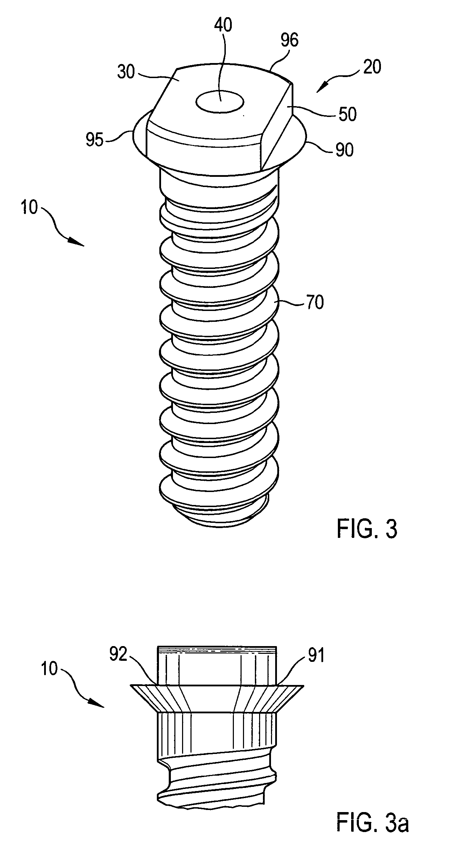

Screw head **20** also has a periphery **96** (identified in **FIG. 1)** which, in the embodiment depicted in **FIG. 1**, has first, second, third, and fourth side surfaces, **50, 51, 60** and **61,** respectively, as identified in **FIG. 1a.** The screw head may have any configuration and thereby the periphery of the screw head may have any number of side surfaces so long as the periphery has opposing first and second side surfaces facing away from each other. As depicted in **FIG. 1,** in a preferred embodiment, opposing first and second side surfaces **50** and **51** are substantially planar and parallel to one another. Parallel first and second side surfaces enable the application of compressive forces to the screw head thus making the screw head able to withstand higher torque with less material (i.e. less thickness) than prior art domed or rounded shape screw heads. Preferably, first and second side surfaces each have a height H of between about 0.5 mm to 5.0 mm and preferably the distance **D**_{**1**} (indicated in **FIG. 1**) between opposing first and second side surfaces is between about 1 mm to 4 mm. Referring to **FIG. 3,** in a preferred embodiment, bone screw **10** comprises opposing first and second ledges **90** and **95,** respectively, positioned below opposing first and second side surfaces **50** and **51** of screw head **20.** Referring to **FIG. 3a,** more preferably, first side surface **50** and first ledge **90** converge to form a first edge **91** and second side surface **51** and second ledge **95** converge to form a second edge **92.** In embodiments where the screw head comprises opposing ledges and the screw head has a circular cross-section, the diameter of the screw head is preferably about 2mm to 10 mm.

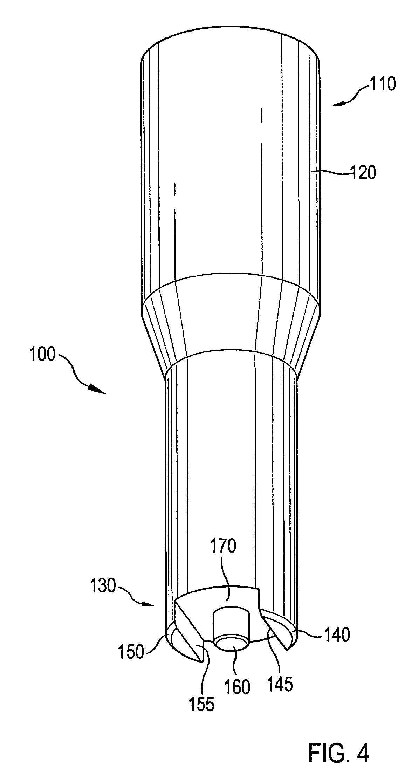

Referring to **FIG. 4,** in an embodiment, a driver **100** of a system of the present invention has a proximal end **110** comprising a handle **120** and a distal end **130.** According to the present invention, distal end **130** is configured to partially encase periphery **96** of screw head **20.** Specifically, distal end **130** comprises opposing first and second side members **140** and **150,** respectively. First member **140** has a first side face **145** and second side member **150** has a second side face **155.** As illustrated in **FIG.** 4, first and second side faces **145** and **155** face one another. First and second side faces **145** and **155** are configured to contact and transmit torque to at least parts of first and second side surfaces **50** and **51** of screw head **20** upon turning of driver **100** thereby avoiding concentration of torque to a relatively small area of bone screw **10** when bone screw **10** is inserted into bone. In a preferred embodiment, first and second side faces **145** and **155** are substantially planar and parallel to one another. Preferably, the maximum thickness T of first and second members **140** and **150** is between about 0.1 mm to 3.0 mm for screws with the aforementioned dimensions, so that the members are sufficiently thick to drive a screw with sufficient torque. As seen in **FIG.** 5, when driver **100** engages screw **10,** periphery **96** of screw head **20** is partially encased by distal end **130** of driver **100** such that first side face **145** of driver **100** faces first side surface **50** of screw head 20 and second side face **155** of driver **100** faces second side surface **51** of screw head **20.** The remaining side surfaces **60** and **61** of periphery **96** are not encased by driver **100.**

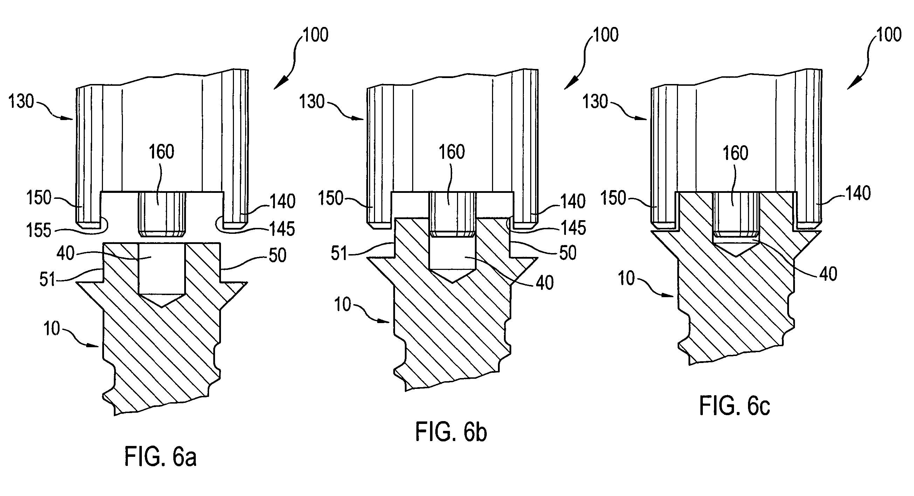

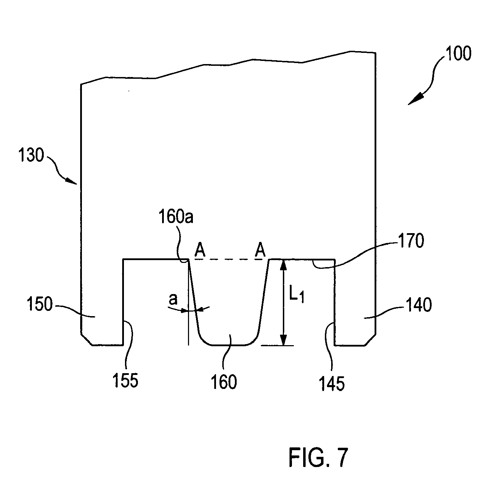

Referring again to **FIG. 4,** according to the present invention, distal end **130** of driver **100** further comprises a metal projection **160** extending from a bottom surface **170** of distal end **130** positioned between opposing first and second side members **140** and **150.** Referring to **FIGS. 6a-c,** metal projection **160** is positioned to be placed in axial alignment with recess **40** of screw 10 and is configured to be received by recess **40** to prevent screw **10** from disengaging from distal end **130** of driver **100** in a direction transverse to the longitudinal axis of the driver. The interaction between the metal projection and the portions of the top surface of the screw head that define the recess is primarily frictional. Although metal projection **160** is illustrated in **FIG.** 4 as being a cylindrical pin, the metal projection may have any cross-sectional configuration so long as the metal projection can be received by the recess of the screw to prevent the screw from laterally disengaging from the distal end of the driver. For example, the metal projection may have any polygonal or non-polygonal cross-sectional shape, such as circular, rectangular, triangular, or elliptical. Referring to **FIG. 7,** preferably metal projection **160** has a length **L**_{**1**}**,** measured from the bottom surface **170** of distal end **130** of driver **100** to the distal most point of metal projection **160** of between about 0.5 mm to 2.0 mm and more preferably about 0.75 mm. Preferably, the cross-sectional area of metal projection **160** taken at base **160a** of metal projection **160** (along line A-A) is between about 0.2mm² and 3.1 mm² and more preferably is about 0.442mm². In embodiments where metal projection **160** has a circular cross-section, the diameter of metal projection **160** taken at base **160a** along line A-A, is between about 0.5mm and 2.0 mm, and more preferably is about 0.75 mm to 0.77 mm. Preferably, the volume of the metal projection is between about 0.1 mm³ and 6.2 mm³. The metal projection **160** may have a constant cross-section or, as illustrated in **FIG. 7**, preferably, metal projection **160** is tapered and narrows distally. Preferably, the tapered distal portion **160b** of metal projection **160** forms an angle a of between about 0.1° to 5° and more preferably about 2.7°. In embodiments where metal projection **160** has a circular cross-section and is tapered, the diameter of metal projection **160** taken at the tip is between about 0.70 and 0.65 mm and more preferably is 0.68 mm. Preferably, the interaction between a metal projection of a driver and a recess of a bone screw of the present invention is such that the when the metal projection is received by the recess, the screw is secured onto the distal end of the driver to releasably lock the bone screw to the driver. Driver **100** may be cannulated along its axis if screw **10** has a bore **80** extending from recess **40** as illustrated in **FIG. 2.**

A bone screw of the present invention can be made of biocompatible and bioabsorbable polymers, copolymers, or polymer mixtures. In certain embodiments of the present invention, the screws are also reinforced with bioabsorbable fibers. Non-limiting examples of known absorbable (biodegradable) polymers which can be used, alone or in mixtures, as raw materials for the screw of the present invention both as matrix (or binder polymers) and/or reinforcement elements include polyglycolide (PGA), glycolide copolymers, glycolide/lactide copolymers (PGA/PLA), glycolide/trimethylene carbonate copolymers (PGA/TMC), stereoisomers and copolymers of PLA, poly-L-lactide (PLLA), poly-D-lactide (PDLA), poly-DL-lactide (PDLLA), L-lactide/DL-lactide copolymers, L-lactide/D-lactide copolymers, copolymers of PLA, lactide/tetramethylene glycolide copolymers, lactide/trimethylene carbonate copolymers, lactide/delta.-valerolactone copolymers, lactide/.epsilon.-caprolactone copolymers, polydepsipeptides (glycine-DL-lactide copolymer), PLA/ethylene oxide copolymers, asymmetrically 3,6-substituted poly-1,4-dioxane-2,4-diones, poly- β -hydroxybutyrate (PHBA), PHBA/ β-hydroxyvalerate copolymers (PHBA/PHVA), poly- β -hydoxypropionate (PHPA), poly-β-dioxanone (PDS), poly-Δ-valerolactone, poly-Δ-caprolactone, methylmethacrylate-N-vinylpyrrolidone copolymers, polyesteramides, polyesters of oxalic acid, polydihydropyranes, polyalkyl-2-cyanoacrylates, polyurethanes (PU), polyvinyl alcohol (PVA), polypeptides, poly- β -maleic acid (PMLA), poly- β -alkanoic acids, polyethylene oxide (PEO), and chitin polymers. The screws of the present invention can be manufactured using either one polymer or a mixture of polymers.

A bone screw of the present invention can be reinforced with polymer fibers or fiber mixtures (such as mixtures of bioabsorbable fibers) which have been made of the above bioabsorbable polymers, copolymers or mixtures thereof. Also other biocompatible fibers, such as carbon fibers, aramide fibers, glass fibers, aluminum oxide fibers, and biostable ceramic fibers may be used as reinforcement for the screws of the present invention. Degradable ceramic fibers, such as tricalcium phosphate fibers and bioactive glass fibers can also be used as reinforcement.

A bone screw of the present invention can also be reinforced through self-reinforcing techniques. A self-reinforced absorbable polymeric material is uniform in its chemical element structure and therefore has good adhesion between the matrix and reinforcement elements. The material has excellent initial mechanical strength properties, such as high tensile, bending or shear strength and toughness, and therefore can be applied favorably in surgical absorbable osteosynthesis devices or as components or parts of such devices, such as screws.

Self-reinforcement means that the polymeric matrix is reinforced with reinforcement elements or materials (such as fibers) which have the same chemical element percentage composition as does the matrix. By applying self-reinforcement principles, the high tensile strength of the fibers can be effectively utilized, when manufacturing macroscopic samples.

When strong oriented fiber structures are bound together with the polymer matrix which has the same chemical element composition as the fibers, a composite structure is obtained which has excellent adhesion between the matrix and reinforcement material and therefore also has excellent mechanical properties.

The material that will form the matrix is subjected to heat and/or pressure in such a way that it allows the development of adhesion between the reinforcement fibers and the matrix. There are alternative methods which can be applied in manufacturing self-reinforced absorbable osteosynthesis materials of the present invention. One method is to mix finely milled polymer powder with fibers, threads or corresponding reinforcement units which are manufactured of the same polymer material or of its isomer with the same chemical element percentage composition, and to heat the mixture under such conditions and using such temperatures that the finely milled particles are softened or melted but the reinforcement unit structures are not significantly softened or melted. When such composition is pressed to the suitable form, the softened or melted particles form a matrix phase that binds the reinforcement units together and when this structure is cooled, a self-reinforced composite with excellent adhesion and mechanical properties is obtained.

The self-reinforced structure of certain embodiments of the present invention can also be obtained by combining together the melt of an absorbable polymer and fibers, threads or corresponding reinforcement elements of the same material, forming the mixture of the polymer melt and reinforcement elements into the desired form and cooling the formed polymer composite rapidly so that the reinforcement elements do not significantly lose their oriented internal structure.

One can also manufacture the self-reinforced absorbable material of the present invention by heating absorbable fibers, threads or corresponding structures in a pressurized mold under such circumstances that at least part of these structures are partially softened or melted on their surface. Under the pressure the softened or melted surface of fibers, threads or corresponding structures are coalesced together and when the mold is cooled, a self-reinforced composite structure is obtained. By a careful control of the heating conditions it is possible to process composite samples where the softened or melted surface regions of fibers, threads or corresponding units are very thin and, therefore, the portion of oriented fiber structure is very high, leading to materials with high tensile, shear, bending and impact strength values.

A bone screw in accordance with the present invention can be manufactured of polymers, copolymers, polymer mixtures and possible degradable and/or biostable reinforcement fibers by various other methods, which are used in plastics technology as well, such as injection molding, extrusion with fibrillation and forming or compression molding wherein the particles are formed from raw materials with aid of heat and/or pressure.

A bone screw in accordance with the present invention also can be manufactured from the above raw materials by so-called solution techniques wherein at least part of the polymer is dissolved or softened by a solvent and the materials or material mixture are affixed to an article through the application of pressure and possibly gentle heat whereupon the dissolved or softened polymer glues the material to the article. The solvent is then removed by evaporating.

A bone screw of the present invention may also contain various additives and adjuvants for facilitating the processability of the material such as stabilizers, antioxidants, or plasticizers; for modifying the properties of thereof such as plasticizers, powdered ceramic materials, or biostable fibers such as aramide or carbon fibers; or for facilitating the manipulation thereof such as colorants.

In a preferred embodiment, a bone screw of the present invention contains some bioactive agent or agents, such as antibiotics, chemotherapeutic agents, wound-healing agents, growth hormones, contraceptive agents, and anticoagulants such as heparin. Such bioactive devices are preferred in clinical applications, since, in addition to mechanical effect, they have beneficial biochemical effects in various tissues.

The metal stabilizing pin of a driver of the present invention can be fabricated from any metallic material, such as, for example, titanium or stainless steel.

A bone screw and driver system of the present invention can be used for bone-to-bone fixation, soft tissue-to-bone fixation, or the fixation of implants, or prostheses to bone and/or to soft tissue. Although a system of the present invention is not limited to any particular orthopedic use, such as system is particularly suited for oral and craniomaxiofacial surgery.

The foregoing description and examples have been set forth merely to illustrate the invention and are not intended as being limiting. Each of the disclosed aspects and embodiments of the present invention may be considered individually or in combination with other aspects, embodiments, and variations of the invention. In addition, unless otherwise specified, none of the steps of the methods of the present invention are confined to any particular order of performance. Modifications of the disclosed embodiments incorporating the spirit and substance of the invention may occur to persons skilled in the art and such modifications are within the scope of the present invention. For example, the dimensions of the bone screw and driver and parts thereof can be scaled up or down with the same relative proportions as disclosed in the specification.

## Claims

1. A bone screw and driver system comprising:
a bioabsorbable bone screw (10) having a screw head (20), the screw head (20) having a top surface (30) integral with a periphery, the top surface (30) defining a recess (40) and the periphery (96) having opposing first (50) and second (51) side surfaces facing away from each other; and
a driver (100) having a distal end (130) configured to partially encase the periphery of the screw head (10), the distal end (130) comprising:
opposing first and second side faces (145, 155) facing towards each other; and
a metal projection (160) positioned between the opposing first and second side faces (145, 155) configured to be received by the recess (40) of the bone screw (10).

2. The system of claim 1, wherein the bone screw (10) comprises opposing first and second ledges (90, 91) positioned below the opposing first and second side surfaces (50, 51) of the screw head.

3. The system of claim 2, wherein the first side surface (50) of the screw head (20) and the first ledge (90) converge to form a first edge (91) and the second side surface of the screw head (20) and the second ledge (95) converge to form a second edge (92).

4. The system of claim 3, wherein the diameter of the screw head (20) is between about 2 millimeters to 10 millimeters.

5. The system of claim 1, wherein each of the opposing first and second side surfaces (50, 51) of the screw head (20) are substantially planar and parallel to one another.

6. The system of claim 1, wherein the distance between the opposing first and second side surfaces (50, 51) is between about 1 millimeters to 4 millimeters.

7. The system of claim 1, wherein the opposing first and second side faces (145, 155) of the driver (100) are substantially planar and parallel to one another.

8. The system of claim 1, wherein the maximum thickness of each of the opposing first and second side faces (50, 51) is between about 0.1 millimeters to 3.0 millimeters.

9. The system of claim 1, wherein the diameter of the recess (40) is at least equal to the depth of the recess (40).

10. The system of claim 1, wherein the bone screw (10) is fabricated from a self-reinforcing material.

11. The system of claim 1, wherein the bone screw (10) comprises an elongated shank (70) extending distally from the screw head (10), the shank (70)defining a bore (80) extending from the recess (40).

12. The system of claim 1, wherein the metal projection (160) comprises a cylindrical pin.

13. The system of claim 1, wherein the metal projection (160) is tapered and narrows distally.

14. The system of claim 1, wherein the interaction between the metal projection (160) and the portion of the top surface (30) defining the recess (40) is primarily frictional.
